# NOUVEAU FASCICULE DE BREVET EUROPEEN

(11) **EP 0 752 244 B2**
(45) Date de publication et mention de la décision concernant l'opposition: **10.01.2001**
(45) Mention de la délivrance du brevet: 04.06.1997
(21) Numéro de dépôt: 96401215.7
(22) Date de dépôt: 06.06.1996
(51) Int. Cl.: A61K 7/06, A61K 7/48, A61K 7/043

(54) **Composition cosmétique contenant un polymère filmogène, procédé de préparation et utilisation**
Kosmetisches Mittel enthaltend ein filmbildendes Polymer, Verfahren zur Herstellung desselben und Verwendung
Cosmetic compositions comprising a film-forming polymer, process for its preparation and use

(30) Priorité: 27.06.1995 FR 9507732
(43) Date de publication de la demande: 08.01.1997
(73) Titulaire: L'OREAL, 75008 Paris (FR)
(72) Inventeur: Mondet, Jean, 93600 Aulnay Sous Bois (FR); Ramin, Roland, 91760 Itteville (FR)
(74) Mandataire: Dodin, Catherine

(56) Documents cités:
- EP-A- 0 291 555
- EP-A- 0 418 469
- EP-A- 0 628 304
- EP-B- 0 644 750
- WO-A-91/15187
- JP-A- 4 103 514
- JP-A- 4 103 515
- JP-A- 5 148 122
- JP-A- 5 155 737
- US-A- 4 158 053
- The Penguin Dictionary of Chemistry, p. 130
- The Penguin Dictionary of Chemistry, p. 168
- The CRC Handbook of Chemistry and Physics, p. C-245
- The Penguin Dictionary of Chemistry, p. 72
- Formulators' Guide : Rhoplex WL for Acrylic Lacquers dated December 1987
- The Merck Index, 9th edition (1976), p. 545

## Description

La présente invention concerne une composition cosmétique comprenant un polymère filmogène, de l'eau et au moins un solvant organique polaire miscible au moins partiellement dans l'eau.

Il est d'usage courant d'utiliser dans de nombreuses formulations cosmétiques, une proportion variable, selon la nature de la formulation, d'au moins une substance filmogène permettant de conférer, par exemple dans le cadre d'une composition capillaire, plus de tenue et de douceur aux cheveux, ou dans le cadre d'un vernis à ongles, un film brillant et dur, adhérant parfaitement sur les ongles.
Il convient toutefois que la substance filmogène présente une bonne affinité vis-à-vis de la kératine des cheveux et des ongles. En d'autres termes, la substance filmogène, une fois la composition appliquée, doit présenter des propriétés de rémanence, c'est à dire être difficilement éliminable de son support par un simple lavage à l'eau ou à l'aide d'un shampooing.

Il est connu de l'état de la technique d'utiliser des compositions dans lesquelles la substance filmogène est en milieu solvant. Toutefois, l'utilisation des solvants organiques volatils étant néfaste pour l'environnement et pour les matières kératiniques, on recherche donc à diminuer, voire à supprimer, la teneur en solvants organiques volatils dans les compositions cosmétiques.
Pour pallier la suppression des solvants organiques dans les compositions filmogènes, il est connu d'utiliser à titre de substance filmogène des dispersions aqueuses de polymères (latex ou pseudolatex), dans lesquelles le polymère, insoluble dans l'eau, est dispersé sous forme de particules. Le polymère se trouvant sous forme particulaire, sa filmification se réalise par coalescence des particules à une vitesse qui dépend notamment des propriétés intrinsèques du polymère. Pour que la filmification entre les particules se fasse rapidement à température ambiante de façon à obtenir des propriétés mécaniques correctes du film déposé, il est préférable que la température de transition vitreuse du polymère soit basse et voisine de 0 °C. Cependant, si l'on veut que le film présente une dureté correcte, notamment pour une utilisation dans un vernis à ongles, il est préférable que le polymère ait une température de transition vitreuse voisine de 30 °C.
Il est difficile d'obtenir une composition filmogène filmifiant rapidement à température ambiante et donnant un film après séchage ayant une bonne dureté, même en utilisant un mélange de plusieurs dispersions aqueuses. D'autre part, l'addition de plastifiants et/ou d'agents de coalescence ne permet pas toujours de parvenir aux meilleures propriétés souhaitées.

On connaît également des compositions filmogènes comprenant un polymère dans un mélange d'eau et de solvant organique.
La demande WO 91/15187 décrit une composition de mise en forme capillaire se présentant sous forme d'émulsion dans laquelle un mélange de polymère et de solvant organique est dispersé dans une phase aqueuse.

La demande JP 63-28411 décrit une composition de vernis à ongles à base de résine, de solvant organique, d'eau et d'un composé miscible à l'eau. La composition se présente sous forme d'émulsion eau-dans-huile, la phase huileuse comprenant le solvant organique et la résine, la phase aqueuse comprenant l'eau et le composé miscible à l'eau.
Ces compositions sous forme d'émulsion ne sèchent pas rapidement, ne s'appliquent pas facilement sur l'ongle et le film obtenu après séchage ne présente pas une excellente tenue.

Le but de la présente invention est de proposer une composition filmogène, qui présente une filmification rapide tout en conservant de bonnes propriétés filmogènes, notamment en donnant un film présentant une bonne dureté.

Ainsi, la présente invention a pour objet une composition cosmétique comprenant un polymère filmogène ionique neutralisé, de l'eau, et au moins un solvant organique polaire au moins partiellement miscible dans l'eau et de point d'ébullition supérieur au point d'ébullition de l'eau, ledit polymère filmogène étant insoluble dans l'eau et soluble dans ledit solvant organique lorsqu'il est à l'état non neutralisé, et étant soluble dans le mélange de solvant organique et d'eau lorsqu'il est neutralisé, le mélange dudit polymère neutralisé, dudit solvant organique et de l'eau constituant une seule phase sous forme d'une solution homogène.

La présente invention a également pour objet un procédé de préparation d'une composition cosmétique, dans lequel on dissout un polymère filmogène ionique non neutralisé, insoluble dans l'eau à l'état non neutralisé, dans un solvant organique polaire au moins partiellement miscible dans l'eau et de point d'ébullition supérieur au point d'ébullition de l'eau, ledit polymère étant soluble dans un mélange d'eau et dudit solvant organique lorsqu'il est neutralisé, puis l'on ajoute à la solution organique de l'eau, l'eau et/ou le solvant organique comprenant au moins un agent de neutralisation du polymère, le mélange dudit polymère neutralisé, dudit solvant organique et de l'eau constituant une seule phase sous forme d'une solution homogène.

L'invention concerne également l'utilisation en cosmétique d'une composition comprenant un polymère filmogène ionique neutralisé, de l'eau, et au moins un solvant organique polaire au moins partiellement miscible dans l'eau et de point d'ébullition supérieur au point d'ébullition de l'eau, ledit polymère filmogène étant insoluble dans l'eau et soluble dans ledit solvant organique lorsqu'il est à l'état non neutralisé, et étant soluble dans le mélange de solvant organique et d'eau lorsqu'il est neutralisé, le mélange dudit polymère neutralisé, dudit solvant organique et de l'eau constituant une seule phase sous forme d'une solution homogène.

Dans la suite de la présente description, on entend par "solvant au moins partiellement miscible dans l'eau", tout solvant dont la solubilité dans l'eau est supérieure ou égale à 10 % en poids, à 25°C.
Par "polymère insoluble dans l'eau", on entend tout polymère dont la solubilité dans l'eau est inférieure ou égale à 2 % en poids, à 25°C.
Par "polymère ionique non neutralisé", on entend tout polymère ionique dont toutes les fonctions ioniques sont libres.
Par "polymère ionique neutralisé", on entend tout polymère dont les fonctions ioniques sont au moins partiellement neutralisées, le taux de neutralisation pouvant aller de 5% à 100%.
Par "une seule phase", on entend une solution homogène.

La composition selon l'invention comprend donc un polymère filmogène ionique neutralisé, de l'eau et un solvant organique polaire au moins partiellement miscible dans l'eau et de point d'ébullition supérieur au point d'ébullition de l'eau.

Le polymère ionique insoluble dans l'eau à l'état non neutralisé peut avoir un poids moléculaire, déterminé par chromatographie d'exclusion stérique, compris entre 500 et 200 000, de préférence entre 1000 et 80 000.
Ainsi, le polymère ionique peut comporter dans sa structure au moins une partie hydrophile et au moins une partie hydrophobe.
La partie hydrophile du polymère peut être constituée de groupements fortement polaires, ayant une forte interaction avec l'eau. Comme groupements fortement polaires, on peut citer le groupement acide carboxylique et ses sels, le groupement acide sulfonique et ses sels, le groupement acide phosphonique et ses sels, des groupements amines primaires, secondaires ou tertiaires et leurs sels, des groupements amines quaternaires, un groupement hydroxyle, un groupement amide hydrophile, un groupement éther ou polyéther, un groupement nitrile, amido, nitro, imido ou mercapto.
De préférence, le polymère ionique comporte des groupements anioniques choisis parmi les groupements acide carboxylique, acide sulfonique et/ou acide phosphonique. Les monomères porteurs de tels groupements peuvent être présents dans le polymère à une teneur comprise entre 3 et 30 % en poids par rapport au poids total du polymère, et de préférence entre 5 et 20 %. Avantageusement, le groupement anionique est un groupement acide carboxylique.
Pour conférer la compatibilité du polymère avec l'eau, c'est-à-dire pour empêcher la précipitation du polymère dans la composition, les groupements anioniques sont de préférence neutralisés avec une base à un taux de neutralisation compris entre 5 % et 100 %. On utilise de préférence une base volatile telle que l'ammoniaque ou la triéthylamine.

Lorsque le polymère comporte des groupements cationiques, ceux-ci peuvent être neutralisés avec un acide à un taux de neutralisation compris entre 5 % et 100 %. On peut utiliser par exemple l'acide acétique.
La partie lipophile du polymère peut être constituée de groupements peu polaires et/ou de groupements hydrophobes. Ces groupements peuvent être par exemple un radical hydrocarboné aliphatique, saturé ou insaturé, linéaire ou ramifié, un radical hydrocarboné aromatique, un radical hydrocarboné cyclique, saturé ou insaturé, un radical fluorocarboné ou un radical organosilicié. Le nombre d'atomes de carbone de ces groupements peut aller de 1 à 30 environ.

De façon avantageuse, le polymère ionique est choisi parmi les polymères radicalaires, les polycondensats et les polymères d'origine naturelle.
Les polymères radicalaires peuvent être choisis parmi les polymères acryliques et les polymères vinyliques.
Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.
Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.
On utilise de préférence des polymères acryliques obtenus par copolymérisation de monomères à insaturation éthylénique contenant des groupements hydrophiles, de préférence de nature non ionique, tels que l'acrylate d'hydroxyéthyle, l'acrylate de 2-hydroxy propyle, le méthacrylate d'hydroxyéthyle, le méthacrylate de 2-hydroxy propyle.
Les polymères vinyliques peuvent résulter de l'homopolymérisation ou de la copolymérisation de monomères choisi parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle.
Selon l'invention, les polycondensats peuvent être choisis parmi les polyesters, les polyesters amides, les polyesters à chaîne grasse, et les résines époxyesters. Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique.

Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylol propane.
Les polyesters amides peuvent être obtenus de manière analogue aux polyesters, par polycondensation de diacides avec des diamines ou des amino alcools. Comme diamine, on peut utiliser l'éthylène diamine, l'hexaméthylènediamine, la méta- ou para-phénylène diamine. Comme aminoalcool, on peut utiliser la monoéthanolamine.
Comme monomère porteur de groupement anionique pouvant être utilisé lors de la polycondensation, on peut citer par exemple l'acide diméthylol propionique, l'acide trimellitique ou un dérivé tel que l'anhydride trimellitique, le sel de sodium de l'acide sulfo-3 pentanediol, le sel de sodium de l'acide 5-sulfo 1,3-benzènedicarboxylique.
Les polyesters à chaîne grasse peuvent être obtenus par l'utilisation de dids à chaîne grasse lors de la polycondensation.
Les résines époxyesters peuvent être obtenues par polycondensation d'acides gras avec un condensat aux extrémités α, ω - diépoxy. De tels polymères sont notamment décrits dans K. KRISHNAMURTI, Progress in organic coatings, 11 (1983), 167-197.
Comme polymère d'origine naturelle, on peut citer la shellac.

Selon l'invention, le solvant organique est au moins partiellement miscible dans l'eau et a un point d'ébullition supérieur à celui de l'eau. Il peut être totalement miscible dans l'eau.
Il est de préférence choisi parmi le propylène glycol, le propylèneglycol monométhyléther, le lactate d'éthyle, le propylèneglycol monopropyléther, le propylèneglycol monotertiobutyléther et le propylèneglycol monométhyléther acétate, seuls ou en mélange.

Selon l'invention, la teneur en polymère filmogène peut être comprise entre 10 % et 70 % en poids par rapport au poids total de la composition.
La teneur en solvant organique miscible au moins partiellement dans l'eau et de point d'ébullition supérieur au point d'ébullition de l'eau peut être comprise entre 4% et 48 % en poids par rapport au poids total de la composition.
La teneur en eau peut être comprise entre 12 % et 75 % , et de préférence entre 40 % et 70 %, en poids par rapport au poids total de la composition.
De préférence, le rapport entre la teneur en eau et la teneur en solvant organique miscible au moins partiellement dans l'eau et de point d'ébullition supérieur au point d'ébullition de l'eau, dans la composition selon l'invention, est compris entre 0,25 et 19, et de préférence entre 0,8 et 17.

La composition peut comprendre en plus au moins un second solvant organique polaire au moins partiellement miscible à l'eau, et ayant un point d'ébullition inférieur ou égal au point d'ébullition de l'eau.
Un tel solvant peut être choisi notamment parmi l'acétone, la méthyléthylcétone, l'isopropanol, l'isobutanol, l'éthanol, le diméthoxyéthane, l'acétate d'amyle, et leurs mélanges.
Il peut être présent dans la composition à une teneur comprise entre 0 % et 40 % en poids par rapport au poids total de la composition.
Le second solvant organique permet de faciliter la mise en oeuvre et en solution du polymère dans le solvant organique miscible au moins partiellement dans l'eau et de point d'ébullition supérieur au point d'ébullition de l'eau. Ainsi, le milieu organique final est plus fluide, la composition s'applique avec plus de facilité et on peut ainsi diminuer la quantité de solvant organique de point d'ébullition supérieur au point d'ébullition de l'eau, présent dans la composition.

La composition peut également comprendre une dispersion aqueuse de polymère.
De préférence, le polymère dispersé ne comporte pas de groupements anioniques ou en comporte à un taux tel qu'il ne représente pas plus de 5 % en poids de monomère porteur de groupement anionique par rapport au poids total de polymère dispersé.
La dispersion aqueuse de polymère peut être un latex ou un pseudolatex. L'expression pseudolatex désigne une suspension constituée de particules généralement sphériques d'un polymère, celles-ci étant obtenues par dispersion du polymère dans une phase aqueuse appropriée. Le latex est également une suspension constituée de particules d'un polymère qui sont obtenues directement par polymérisation d'un ou plusieurs monomères dans une phase aqueuse appropriée.
Le polymère en dispersion aqueuse peut être choisi parmi les polyuréthannes, les polymères acryliques, les alkydes, les polyesters et les polyesteramides. De préférence, le polymère en dispersion aqueuse a une température de transition vitreuse supérieure ou égale à 10 °C.
Avantageusement, le polymère en dispersion aqueuse est un polymère acrylique tel qu'un copolymère de méthacrylate de méthyle ou de styrène, avec un acrylate ou méthacrylate de méthyle, d'éthyle ou d'éthyl 2-hexyle.
Le polymère en dispersion aqueuse peut être présent dans la composition selon l'invention à une teneur comprise entre 30 % et 90 % en poids de matière sèche par rapport au poids total de la composition, et de préférence entre 50 % et 80 %. Lorsque la composition selon l'invention comprend une telle dispersion aqueuse de polymère, on observe une filmification plus rapide de ladite composition, qui conserve ses propriétés mécaniques et de résistance à l'eau. Le film obtenu est plus brillant, plus adhérent et présente une bonne dureté de surface. Une telle composition convient particulièrement pour une utilisation comme vernis à ongles.

La composition selon l'invention peut contenir des adjuvants couramment utilisés en cosmétique. On peut citer à titre d'exemple d'adjuvants les colorants, les pigments, les nacres, les laques, les agents anti-UV, les agents épaississants, les parfums, les agents anti-mousse, les agents tensioactifs, les agents bactéricides. Bien entendu l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

Ainsi, lors de l'utilisation de la composition selon l'invention, on constate que le solvant organique de point d'ébullition supérieur au point d'ébullition de l'eau ne s'évapore totalement qu'après l'évaporation de l'eau et sert donc de solvant au polymère lors du stade final de la filmification de la composition.
On obtient les mêmes propriétés de brillance et de dureté du film que celles obtenues avec une composition filmogène en milieu solvant, et la filmification totale de la composition est plus rapide qu'avec une dispersion aqueuse de polymère. En particulier, le film obtenu après séchage présente une bonne brillance et une bonne adhérence, ainsi qu'une bonne tenue.
Lors de l'application de la composition selon l'invention sur l'ongle, on constate une bonne mouillabilité de l'ongle ce qui facilite l'application de la composition.

La composition de l'invention peut se présenter sous forme d'une composition capillaire, par exemple à effet coiffant ou sous forme d'une composition de maquillage telle qu'un vernis à ongles ou un mascara, ou encore sous forme de composition de soin de la peau telle qu'un masque de beauté ou une lotion de soin pour le visage exerçant des effets tenseurs sur la peau. Elle peut se présenter également sous forme d'une composition de soin des ongles.

On va maintenant donner des exemples illustrant la présente invention sans toutefois la limiter.

### Exemple 1:

On a préparé une base incolore de produit de soin des ongles ayant la composition suivante :

| | |
|---|---|
| - Résine alkyde à 75% de matière sèche (MS) dans un mélange de butyl glycol et de 2-butanol (50/50) vendue sous la dénomination WORLEESOL 60 A par la société WORLLE | 13g MS |
| - Dispersion aqueuse acrylique à 42 % de matière sèche vendue sous la dénomination PRIMAL WL81 K par ROHM et HAAS | 13 g MS |
| - Isopropanol | 5,5 g |
| - Ether monométhylique de propylèneglycol | 8,5 g |
| - Triéthylamine | 1,2 g |
| -Additifs | 0,5 g |
| - Actifs | 0,5 g |
| - Eau | qsp 100 g |

On mélange sous agitation la résine alkyde, l'isopropanol et l'éther monométhylique de propylèneglycol, puis on ajoute les additifs et la triéthylamine. On ajoute ensuite l'eau et les actifs. Enfin, on additionne la dispersion acrylique. La base de soin s'étale facilement sur l'ongle et filmifie rapidement. Elle donne après séchage un film résistant et dur. Cette base peut être recouverte par un vernis coloré.

### Exemple 2:

On a préparé, selon le mode opératoire de l'exemple 1, un vernis à ongles ayant la composition suivante :

| | |
|---|---|
| - Résine alkyde à 75 % de matière sèche dans un mélange de butyl glycol et de 2-butanol (50/50) (WORLEESOL 60 A de WORLLE) | 15 g MS |
| - Dispersion aqueuse acrylique à 42 % de matière sèche (PRIMAL WL81 K de ROHM et HAAS) | 15 g MS |
| - Isopropanol | 6,5 g |
| - Ether monoéthylique de propylène glycol | 4,5 g |
| - Ether monobutylique de propylène glycol | 8,5 g |
| - Triéthylamine | 1,3 g |
| - Agent rhéologique | 0,5 g |
| - Additifs | 0,5 g |
| - Pigments | 0,7 g |
| - Eau | qsp 100 g |

On obtient un vernis qui s'applique facilement sur l'ongle. Après séchage, le film obtenu est lisse et homogène.

### Exemple 3:

On a préparé un vernis à ongles ayant la composition suivante :

| | |
|---|---|
| - Résine alkyde à 75 % de matière sèche dans un mélange de butyl glycol et de 2-butanol (50/50) (WORLEESOL 60 A de WORLLE) | 30 g MS |
| - isopropanol | 6,5 g |
| - Triéthylamine | 2,6 g |
| - Ethanol | 13 g |
| - Agent rhéologique | 0,5 g |
| - Additifs | 0,5 g |
| - Pigments | 0,7 g |
| - Eau | qsp 100 g |

On obtient un vernis qui s'applique facilement sur l'ongle. Après séchage, le film obtenu est lisse et homogène.

## Revendications

1. Composition cosmétique comprenant un polymère filmogène ionique neutralisé, de l'eau, et au moins un solvant organique polaire au moins partiellement miscible dans l'eau et de point d'ébullition supérieur au point d'ébullition de l'eau, ledit polymère filmogène étant insoluble dans l'eau et soluble dans ledit solvant organique lorsqu'il est à l'état non neutralisé, et étant soluble dans le mélange de solvant organique et d'eau lorsqu'il est neutralisé, le mélange dudit polymère neutralisé, dudit solvant organique et de l'eau constituant une seule phase sous forme d'une solution homogène.

2. Composition cosmétique selon la revendication 1, caractérisée par le fait que le polymère filmogène ionique comporte une partie hydrophile constituée de groupements fortement polaires choisis parmi le groupement acide carboxylique et ses sels, le groupement acide sulfonique et ses sels, le groupement acide phosphonique et ses sels, des groupements amines primaires, secondaires ou tertiaires et leurs sels, des groupements amines quaternaires, un groupement hydroxyle, un groupement amide hydrophile, un groupement éther ou polyéther, un groupement nitrile, amido, nitro, imido ou mercapto.

3. Composition cosmétique selon l'une des revendications précédentes, caractérisée par le fait que le polymère filmogène ionique comporte des groupements anioniques choisis parmi les groupements acide carboxylique, acide sulfonique et acide phosphonique, neutralisés à un taux compris entre 5 % et 100 %.

4. Composition cosmétique selon la revendication 3, caractérisée par le fait que le polymère filmogène ionique comporte 3 à 30 % en poids de monomères porteurs de groupements anioniques par rapport au poids total du polymère.

5. Composition cosmétique selon l'une des revendications 3 à 4, caractérisée par le fait le groupement anionique est un groupement acide carboxylique.

6. Composition cosmétique selon l'une quelconque des revendications précédentes, caractérisée par le fait que le polymère filmogène ionique comporte une partie lipophile constituée de groupements peu polaires et/ou de groupements hydrophobes, choisis parmi un radical hydrocarboné aliphatique, saturé ou insaturé, linéaire ou ramifié, un radical hydrocarboné aromatique, un radical hydrocarboné cyclique, saturé ou insaturé, un radical fluorocarboné et un radical organosilicié.

7. Composition cosmétique selon la revendication 1, caractérisée par le fait que le polymère filmogène ionique est choisi parmi les polymères radicalaires, les polycondensats et les polymères d'origine naturelle.

8. Composition cosmétique selon la revendication 7, caractérisée par le fait que les polymères radicalaires sont choisis parmi les polymères acryliques et les polymères vinyliques.

9. Composition cosmétique selon la revendication 7, caractérisée par le fait que les polycondensats sont choisis parmi les polyesters, les polyesters amides, les polyesters à chaînes grasses, les résines époxyesters.

10. Composition cosmétique selon l'une quelconque des revendications précédentes, caractérisée par le fait que le polymère filmogène ionique a un poids moléculaire, déterminé par chromatographie d'exclusion stérique, compris entre 500 et 200 000.

11. Composition cosmétique selon l'une quelconque des revendications précédentes, caractérisée par le fait que le solvant organique ayant un point d'ébullition supérieur à celui de l'eau est choisi parmi le propylène glycol, le propylèneglycol monométhyléther, le lactate d'éthyle, le propylèneglycol monopropyléther, le propylèneglycol monotertiobutyl-éther, le propylèneglycol monométhyléther acétate, et leurs mélanges.

12. Composition cosmétique selon l'une quelconque des revendications précédentes, caractérisée par le fait que la composition a une teneur en eau comprise entre 12 % et 75 % en poids par rapport au poids total de la composition.

13. Composition cosmétique selon l'une quelconque des revendications précédentes, caractérisée par le fait que la teneur en solvant organique miscible dans l'eau et de point d'ébullition supérieur au point d'ébullition de l'eau est comprise entre 4% et 48 % en poids par rapport au poids total de la composition.

14. Composition cosmétique selon l'une quelconque des revendications précédentes, caractérisée par le fait que la teneur en polymère filmogène est comprise entre 10 % et 70 % en poids par rapport au poids total de la composition.

15. Composition cosmétique selon l'une quelconque des revendications précédentes, caractérisée par le fait qu'elle comprend en plus au moins un solvant organique polaire au moins partiellement miscible à l'eau et ayant un point d'ébullition inférieur ou égal au point d'ébullition de l'eau.

16. Composition cosmétique selon la revendication 15, caractérisée par le fait que le solvant organique polaire miscible à l'eau ayant un point d'ébullition inférieur ou égal au point d'ébullition de l'eau est choisi parmi l'acétone, la méthyléthylcétone, l'isopropanol, l'isobutanol, l'éthanol, le diméthoxyéthane, l'acétate d'amyle, et leurs mélanges.

17. Composition cosmétique selon l'une quelconque des revendications précédentes, caractérisée par le fait que la composition comprend en plus une dispersion aqueuse de polymère.

18. Composition cosmétique selon la revendication 17, caractérisée par le fait que le polymère en dispersion aqueuse comporte de 0 à 5 % en poids de monomère porteur de groupement anionique par rapport au poids total du polymère dispersé.

19. Composition cosmétique selon l'une des revendications 17 ou 18, caractérisée par le fait le polymère en dispersion aqueuse est choisi parmi les polyuréthannes, les polymères acryliques, les alkydes, les polyesters et les polyesteramides.

20. Composition cosmétique selon l'une des revendications 17 à 19, caractérisée par le fait que le polymère en dispersion aqueuse est un polymère acrylique tel qu'un copolymère de méthacrylate de méthyle ou de styrène, avec un acrylate ou méthacrylate de méthyle, d'éthyle ou d'éthyl 2-hexyle.

21. Composition cosmétique selon l'une des revendications 17 à 20, caractérisée par le fait que le polymère en dispersion aqueuse a une température de transition vitreuse supérieure ou égale à 10 °C.

22. Composition cosmétique selon l'une quelconque des revendications précédentes, caractérisée par le fait qu'elle comprend au moins un adjuvant cosmétique.

23. Composition cosmétique selon l'une quelconque des revendications précédentes, caractérisée par le fait qu'elle se présente sous forme d'une composition capillaire, d'une composition de maquillage et notamment un vernis à ongles ou un mascara, d'une composition de soin de la peau ou d'une composition de soin des ongles.

24. Procédé de préparation d'une composition cosmétique, selon l'une des revendications 1 à 23, dans lequel
. on dissout un polymère filmogène ionique non neutralisé, insoluble dans l'eau à l'état non neutralisé, dans un solvant organique polaire au moins partiellement miscible dans l'eau et de point d'ébullition supérieur au point d'ébullition de l'eau, ledit polymère étant soluble dans un mélange d'eau et dudit solvant organique lorsqu'il est neutralisé, puis
. l'on ajoute à la solution organique de l'eau, l'eau et/ou le solvant organique comprenant au moins un agent de neutralisation du polymère, et
. le mélange dudit polymère neutralisé, dudit solvant organique et de l'eau constituant une seule phase sous forme d'une solution homogène.

25. Procédé selon la revendication 24, caractérisé par le fait que le polymère comporte des groupements anioniques et est neutralisé par une base volatile.

26. Utilisation en cosmétique d'une composition comprenant un polymère filmogène ionique neutralisé, de l'eau, et au moins un solvant organique polaire au moins partiellement miscible dans l'eau et de point d'ébullition supérieur au point d'ébullition de l'eau, ledit polymère filmogène étant insoluble dans l'eau et soluble dans ledit solvant organique lorsqu'il est à l'état non neutralisé, et étant soluble dans le mélange de solvant organique et d'eau lorsqu'il est neutralisé, le mélange dudit polymère neutralisé, dudit solvant organique et de l'eau constituant une seule phase sous forme d'une solution homogène.

## Patentansprüche

1. Kosmetische Zusammensetzung, die ein neutralisiertes ionisches filmbildendes Polymer, Wasser und mindestens ein polares organisches Lösungsmittel, das mindestens teilweise mit Wasser mischbar ist und einen Siedepunkt oberhalb des Siedepunkts von Wasser aufweist, enthält, wobei das filmbildende Polymer in Wasser unlöslich und in dem organischen Lösungsmittel löslich ist, wenn es nicht neutralisiert ist, und in dem Gemisch aus dem organischem Lösungsmittel und Wasser löslich ist, wenn es neutralisiert ist, und wobei das Gemisch aus dem neutralisierten Polymer, dem organischen Lösungsmittel und Wasser eine einzige Phase in Form einer homogenen Lösung bildet.

2. Kosmetische Zusammensetzung nach Anspruch 1, dadurch gekennzeichnet, daß das ionische filmbildende Polymer einen hydrophilen Teil aufweist, der aus stark polaren Gruppen gebildet ist, die unter der Carboxygruppe und deren Salzen, der Sulfonsäuregruppe und deren Salzen, der Phosphonsäuregruppe und deren Salzen, primären, sekundären und tertiären Amingruppen und deren Salzen, quaternären Amingruppen, der Hydroxygruppe, hydrophilen Amidgruppen, Ether- und Polyethergruppen, einer Nitril-, Amido-, Nitro-, Imido- oder Mercaptogruppe ausgewählt sind.

3. Kosmetische Zusammensetzung nach einem der vorhergehenden Ansprüche, dadurch gekennzeichnet, daß das ionische filmbildende Polymer anionische Gruppen enthält, die unter der Carboxygruppe, der Sulfonsäuregruppe und der Phosphonsäuregruppe ausgewählt sind, die zu 5 bis 100 % neutralisiert sind.

4. Kosmetische Zusammensetzung nach Anspruch 3, dadurch gekennzeichnet, daß das ionische filmbildende Polymer 3 bis 30 Gew.-% Monomere, die eine oder mehrere anionische Gruppen tragen, bezogen auf das Gesamtgewicht des Polymers, enthält.

5. Kosmetische Zusammensetzung nach einem der Ansprüche 3 und 4, dadurch gekennzeichnet, daß die anionische Gruppe eine Carboxygruppe ist.

6. Kosmetische Zusammensetzung nach einem der vorhergehenden Ansprüche, dadurch gekennzeichnet, daß das ionische filmbildende Polymer einen lipophilen Teil aufweist, der aus wenig polaren und/oder hydrophoben Gruppen gebildet ist, die unter gesättigten oder ungesättigten, geradkettigen oder verzweigten aliphatischen Kohlenwasserstoffgruppen, aromatischen Kohlenwasserstoffgruppen, gesättigten oder ungesättigten cyclischen Kohlenwasserstoffgruppen, Fluorkohlenstoffgruppen und siliciumorganischen Gruppen ausgewählt sind.

7. Kosmetische Zusammensetzung nach Anspruch 1, dadurch gekennzeichnet, daß das ionische filmbildende Polymer unter radikalischen Polymeren, Polykondensaten und Polymeren natürlicher Herkunft ausgewählt ist.

8. Kosmetische Zusammensetzung nach Anspruch 7, dadurch gekennzeichnet, daß die radikalischen Polymere unter Acrylpolymeren und Vinylpolymeren ausgewählt sind.

9. Kosmetische Zusammensetzung nach Anspruch 7, dadurch gekennzeichnet, daß die Polykondensate unter Polyestern, Polyesteramiden, Polyestern mit Fettketten, Epoxyesterharzen ausgewählt sind.

10. Kosmetische Zusammensetzung nach einem der vorhergehenden Ansprüche, dadurch gekennzeichnet, daß das ionische filmbildende Polymer ein Molekulargewicht, das durch sterische Ausschlußchromatographie bestimmt wird, von 500 bis 200 000 aufweist.

11. Kosmetische Zusammensetzung nach einem der vorhergehenden Ansprüche, dadurch gekennzeichnet, daß das organische Lösungsmittel, das einen Siedepunkt oberhalb des Siedepunkts von Wasser aufweist, unter Propylenglykol, Propylenglykolmonomethylether, Ethyllactat, Propylenglykolmonopropylether, Propylenglykolmono-*tert.*-butylether, Propylenglykolmonomethyletheracetat und deren Gemischen ausgewählt ist.

12. Kosmetische Zusammensetzung nach einem der vorhergehenden Ansprüche, dadurch gekennzeichnet, daß die Zusammensetzung einen Wassergehalt von 12 bis 75 Gew.-%, bezogen auf das Gesamtgewicht der Zusammensetzung, aufweist.

13. Kosmetische Zusammensetzung nach einem der vorhergehenden Ansprüche, dadurch gekennzeichnet, daß der Gehalt an organischem Lösungsmittel, das mit Wasser mischbar ist und einen Siedepunkt oberhalb des Siedepunkts von Wasser aufweist, 4 bis 48 Gew.-%, bezogen auf das Gesamtgewicht der Zusammensetzung, beträgt.

14. Kosmetische Zusammensetzung nach einem der vorhergehenden Ansprüche, dadurch gekennzeichnet, daß der Gehalt an filmbildendem Polymer 10 bis 70 Gew.-%, bezogen auf das Gesamtgewicht der Zusammensetzung, beträgt.

15. Kosmetische Zusammensetzung nach einem der vorhergehenden Ansprüche, dadurch gekennzeichnet, daß sie außerdem mindestens ein polares organisches Lösungsmittel enthält, das mindestens teilweise mit Wasser mischbar ist und einen Siedepunkt aufweist, der unterhalb des Siedepunkts von Wasser liegt oder dem Siedepunkt von Wasser entspricht.

16. Kosmetische Zusammensetzung nach Anspruch 15, dadurch gekennzeichnet, daß das polare organische Lösungsmittel, das mit Wasser mischbar ist und einen Siedepunkt aufweist, der unterhalb des Siedepunkts von Wasser liegt oder dem Siedepunkt von Wasser entspricht, unter Aceton, Methylethylketon, Isopropanol, Isobutanol, Ethanol, Dimethoxyethan, Amylacetat und deren Gemischen ausgewählt ist.

17. Kosmetische Zusammensetzung nach einem der vorhergehenden Ansprüche, dadurch gekennzeichnet, daß die Zusammensetzung außerdem eine wäßrige Polymerdispersion enthält.

18. Kosmetische Zusammensetzung nach Anspruch 17, dadurch gekennzeichnet, daß das in wäßriger Dispersion vorliegende Polymer 0 bis 5 Gew.-% Monomer, das eine oder mehrere anionische Gruppe tragt, bezogen auf das Gesamtgewicht des dispergierten Polymers, enthält.

19. Kosmetische Zusammensetzung nach Anspruch 17 oder 18, dadurch gekennzeichnet, daß das in wäßriger Dispersion vorliegende Polymer unter Polyurethanen, Acrylpolymeren, Alkyden, Polyestern und Polyesteramiden ausgewählt ist.

20. Kosmetische Zusammensetzung nach einem der Ansprüche 17 bis 19, dadurch gekennzeichnet, daß das in wäßriger Dispersion vorliegende Polymer ein Acrylpolymer, wie z.B. ein Copolymer aus Methylmethacrylat oder Styrol mit einem Methylacrylat oder Methylmethacrylat, Ethylacrylat oder Ethylmethacrylat, 2-Ethylhexylacrylat oder 2-Ethylhexylmethacrylat ist.

21. Kosmetische Zusammensetzung nach einem der Ansprüche 17 bis 20, dadurch gekennzeichnet, daß das in wäßriger Dispersion vorliegende Polymer eine Glasübergangstemperatur aufweist, die bei 10 °C oder darüber liegt.

22. Kosmetische Zusammensetzung nach einem der vorhergehenden Ansprüche, dadurch gekennzeichnet, daß sie mindestens einen kosmetischen Hilfsstoff enthält.

23. Kosmetische Zusammensetzung nach einem der vorhergehenden Ansprüche, dadurch gekennzeichnet, daß sie in Form einer Zusammensetzung zur Haarbehandlung, einer Schminkzusammensetzung und insbesondere in Form eines Nagellacks oder einer Wimperntusche, einer Zusammensetzung für die Hautpflege oder einer Zusammensetzung für die Nagelpflege vorliegt.

24. Verfahren zur Herstellung einer kosmetischen Zusammensetzung nach einem der Ansprüche 1 bis 23, bei dem
- ein nicht neutralisiertes ionisches filmbildendes Polymer, das im nicht neutralisierten Zustand in Wasser unlöslich ist, in einem polaren organischen Lösungsmittel, das mindestens teilweise mit Wasser mischbar ist und einen Siedepunkt oberhalb des Siedepunkts von Wasser aufweist, gelöst wird, wobei das Polymer in einem Gemisch aus Wasser und dem obengenannten organischen Lösungsmittel löslich ist, wenn es neutralisiert ist,
- zu der organischen Lösung Wasser, das Wasser und/oder das organische Lösungsmittel, das mindestens ein Mittel zur Neutralisation des Polymers enthält, gegeben wird und
- das Gemisch aus dem neutralisierten Polymer, dem organischen Lösungsmittel und Wasser eine einzige Phase in Form einer homogenen Lösung bilden.

25. Verfahren nach Anspruch 24, dadurch gekennzeichnet, daß das Polymer anionische Gruppen aufweist und mit einer flüchtigen Base neutralisiert wird.

26. Verwendung einer Zusammensetzung in der Kosmetik, die ein neutralisiertes ionisches filmbildendes Polymer, Wasser und mindestens ein polares organisches Lösungsmittel, das mindestens teilweise mit Wasser mischbar ist und einen Siedepunkt oberhalb des Siedepunkts von Wasser aufweist, enthält, wobei das filmbildende Polymer in Wasser unlöslich und dem organischen Lösungsmittel löslich ist, wenn es nicht neutralisiert ist, und in dem Gemisch aus organischem Lösungsmittel und Wasser löslich ist, wenn es neutralisiert ist, und wobei das Gemisch aus neutralisiertem Polymer, organischem Lösungsmittel und Wasser eine einzige Phase in Form einer homogenen Lösung bildet.

## Claims

1. Cosmetic composition comprising a neutralized ionic film-forming polymer, water and at least one polar organic solvent which is at least partially water-miscible and of higher boiling point than the boiling point of water, the said, film-forming polymer being insoluble in water and soluble in the said organic solvent when it is in the non-neutralized state, and being soluble in the mixture of organic solvent and water when it is neutralized, the mixture of the said neutralized polymer, the said organic solvent and water constituting a single phase in the form of a homogeneous solution.

2. Cosmetic composition according to Claim 1, characterized in that the ionic film-forming polymer includes a hydrophilic part consisting of highly polar groups chosen from the carboxylic acid group and salts thereof, the sulphonic acid group and salts thereof, the phosphonic acid group and salts thereof, primary, secondary or tertiary amine groups and salts thereof, quaternary amine groups, a hydroxyl group, a hydrophilic amide group, an ether or polyether group or a nitrile, amido, nitro, imido or mercapto group.

3. Cosmetic composition according to either of the preceding claims, characterized in that the ionic film-forming polymer includes anionic groups chosen from carboxylic acid, sulphonic acid and phosphonic acid groups, neutralized to a degree of between 5 % and 100 %.

4. Cosmetic composition according to Claim 3, characterized in that the ionic film-forming polymer includes 3 to 30 % by weight of monomers bearing anionic groups relative to the total weight of the polymer.

5. Cosmetic composition according to either of Claims 3 and 4, characterized in that the anionic group is a carboxylic acid group.

6. Cosmetic composition according to any one of the preceding claims, characterized in that the ionic film-forming polymer includes a lipophilic part consisting of relatively non-polar groups and/or of hydrophobic groups, chosen from a linear or branched, saturated or unsaturated aliphatic hydrocarbon radical, an aromatic hydrocarbon radical, a saturated or unsaturated cyclic hydrocarbon radical, a fluorocarbon radical and an organosilicon radical.

7. Cosmetic composition according to Claim 1, characterized in that the ionic film-forming polymer is chosen from radical polymers, polycondensates and polymers of natural origin.

8. Cosmetic composition according to Claim 7, characterized in that the radical polymers are chosen from acrylic polymers and vinyl polymers.

9. Cosmetic composition according to Claim 7, characterized in that the polycondensates are chosen from polyesters, polyester amides, fatty-chain polyesters and epoxyester resins.

10. Cosmetic composition according to any one of the preceding claims, characterized in that the ionic film-forming polymer has a molecular weight, determined by steric exclusion chromatography, of between 500 and 200,000.

11. Cosmetic composition according to any one of the preceding claims, characterized in that the organic solvent having a boiling point higher than that of water is chosen from propylene glycol, propylene glycol monomethyl ether, ethyl lactate, propylene glycol monopropyl ether, propylene glycol mono-tert-butyl ether and propylene glycol monomethyl ether acetate, and mixtures thereof.

12. Cosmetic composition according to any one of the preceding claims, characterized in that the composition has a water content of between 12 % and 75 % by weight relative to the total weight of the composition.

13. Cosmetic composition according to any one of the preceding claims, characterized in that the content of organic solvent which is water-miscible and of boiling point higher than the boiling point of water is between 4 % and 48 % by weight relative to the total weight of the composition.

14. Cosmetic composition according to any one of the preceding claims, characterized in that the content of film-forming polymer is between 10 % and 70 % by weight relative to the total weight of the composition.

15. Cosmetic composition according to any one of the preceding claims, characterized in that it additionally comprises at least one polar organic solvent which is at least partially water-miscible and which has a boiling point below or equal to the boiling point of water.

16. Cosmetic composition according to Claim 15, characterized in that the polar organic solvent which is water-miscible and which has a boiling point below or equal to the boiling point of water is chosen from acetone, methyl ethyl ketone, isopropanol, isobutanol, ethanol, dimethoxyethane and amyl acetate, and mixtures thereof.

17. Cosmetic composition according to any one of the preceding claims, characterized in that the composition also comprises an aqueous polymer dispersion.

18. Cosmetic composition according to Claim 17, characterized in that the polymer in aqueous dispersion includes from 0 to 5 % by weight of anionic-group-bearing monomer relative to the total weight of the dispersed polymer.

19. Cosmetic composition according to either of Claims 17 and 18, characterized in that the polymer in aqueous dispersion is chosen from polyurethanes, acrylic polymers, alkyds, polyesters and polyester amides.

20. Cosmetic composition according to one of Claims 17 to 19, characterized in that the polymer in aqueous dispersion is an acrylic polymer such as a copolymer of methyl methacrylate or styrene, with a methyl, ethyl or 2-ethylhexyl acrylate or methacrylate.

21. Cosmetic composition according to one of Claims 17 to 20, characterized in that the polymer in aqueous dispersion has a glass transition temperature of above or equal to 10°C.

22. Cosmetic composition according to any one of the preceding claims, characterized in that it comprises at least one cosmetic adjuvant.

23. Cosmetic composition according to any one of the preceding claims, characterized in that it is in the form of a hair composition, a make-up composition and, in particular, a nail varnish or a mascara, a skin care composition or a nail care composition.

24. Process for the preparation of a cosmetic composition, according to one of Claims 1 to 23, in which
. a non-neutralized ionic film-forming polymer, which is water-insoluble in the non-neutralized state, is dissolved in a polar organic solvent which is at least partially water-miscible and of higher boiling point than the boiling point of water, the said polymer being soluble in a mixture of water and the said organic solvent when it is neutralized, then
. water is added to the organic solution, the water and/or the organic solvent comprising at least one agent for neutralization of the polymer, and
. the mixture of the said neutralized polymer, the said organic solvent and the water constituting a single phase in the form of a homogeneous solution.

25. Process according to Claim 24, characterized in that the polymer includes anionic groups and is neutralized with a volatile base.

26. Use in cosmetics of a composition comprising a neutralized ionic film-forming polymer, water and at least one polar organic solvent which is at least partially water-miscible and of higher boiling point than the boiling point of water, the said film-forming polymer being insoluble in water and soluble in the said organic solvent when it is in the non-neutralized state, and being soluble in the mixture of organic solvent and water when it is neutralized, the mixture of the said neutralized polymer, the said organic solvent and the water constituting a single phase in the form of a homogeneous solution.
